# EUROPÄISCHE PATENTANMELDUNG

(11) **EP 4 215 916 A1**
(43) Veröffentlichungstag der Anmeldung: **26.07.2023**
(21) Anmeldenummer: 22152398.8
(22) Anmeldetag: 20.01.2022
(51) Int. Cl.: G01N 33/531, C07K 14/00, G01N 33/569

(54) **TEST ZUM NACHWEIS VON ANTIKÖRPERN GEGEN LEISHMANIEN**

(71) Anmelder: Philipps-Universität Marburg, 35037 Marburg (DE)
(72) Erfinder: Steinhoff, Ulrich, 35037 Marburg (DE); Mahdavi, Rouzbeh, 35037 Marburg (DE)
(74) Vertreter: Stumpf, Peter

(57) **Zusammenfassung**

Die Erfindung betrifft ein Verfahren zum Nachweis von Antikörpern gegen Leishmanien in einer biologischen Probe und umfasst folgende Schritte:
(i) Bereitstellung einer biologischen Probe eines Menschen oder eines Hundes;
(ii) Bereitstellung des auf einer Unterlage gebundenen Testantigens mit der SEQ ID NO. 8, das 8,3 repetitive Sequenzen aufweist und das von Antikörpern gegen verschiedene Leishmanienstämme erkannt und gebunden werden kann;
(iii) Inkubation der biologischen Probe aus Schritt (i) mit dem Testantigen aus Schritt (ii), so dass Antigen-Antikörper-Komplexe gebildet werden, wenn in der biologischen Probe aus Schritt (i) Antikörper gegen mindestens einen Leishmanienstamm enthalten sind;
(iv) Entfernung von nicht an das Testantigen aus Schritt (ii) gebundene Antikörper und sonstigem Material aus der biologischen Probe aus Schritt (i) in einem ersten Waschschritt;
(v) Zugabe eines human-spezifischen Sekundärantikörpers, wenn die biologische Probe aus Schritt (i) von einem Mensch stammt oder Zugabe eines canin-spezifischen Sekundärantikörpers, wenn die biologische Probe aus Schritt (i) von einem Hund stammt, wobei der jeweilige Sekundärantikörper durch ein Mittel zur Sichtbarmachung einer Bindung des Sekundärantikörpers nachweisbar ist, so dass der jeweilige Sekundärantikörper an die Antigen-Antikörper-Komplexe aus Schritt (iii) bindet;
(vi) Entfernung von nicht gebundenen Sekundärantikörpern aus Schritt (v) in einem zweiten Waschschritt;
(vii) Sichtbarmachung der Bindung des Sekundärantikörpers an den Antigen-Antikörper-Komplexen aus Schritt (iii) durch Zugabe eines geeigneten Mittels.

Zusätzlich betrifft die Erfindung einen Test zur Durchführung des Verfahrens, die Herstellung des Testantigens und das Testantigen mit der SEQ ID NO. 8.

## Beschreibung

Die Erfindung betrifft ein Verfahren zum Nachweis von Antikörper gegen Leishmanien bei Menschen und Hunden (Patienten). Für das neue serologische Verfahren wird ein neues Testantigen verwendet. Durch den Nachweis von Antikörper gegen Leishmanien wird die Erkrankung Leishmaniose (englisch: Leishmaniasis) diagnostiziert. Das neue Testantigen bindet spezifisch an Antikörper, die ein Patient nach einer Infektion mit Leishmanien gebildet hat. Diese Testantigen-Antikörper-Bindung wird anschließend mit einem geeigneten, dem Fachmann bekannten Verfahren sichtbar gemacht, beispielsweise durch einen markierten Sekundärantikörper. Wenn sich ein Patient mit Leishmanien infiziert hat und an Leishmaniose erkrankt ist, dann hat dieser Patient auch Antikörper gegen Leishmanien gebildet, welche mit einem serologischen Verfahren nachweisbar sind. Die Erfindung betrifft ein neues Testantigen, mit dem speziesübergreifend (also bei Menschen und Hunden als Patienten) und stammesübergreifend (also bei Infektionen mit verschiedenen Leishmanienstämmen bzw. Leishmanienarten) Antikörper gegen Leishmanien nachgewiesen werden können. Insbesondere betrifft die Erfindung ein Verfahren, das mittels eines point-of-care-Tests (POCT, patientennahe Labordiagnostik, Schnelltest) zur direkten Anwendung an Menschen und Hunden in den von Leishmaniose betroffenen Ländern durchführbar ist.

Die Erfindung betrifft ebenfalls die Herstellung dieses neuen Testantigens sowie einen neuen serologischen Test (point-of-care-Test, POCT, patientennahe Labordiagnostik, Schnelltest) zum serologischen Nachweis von Antikörper gegen Leishmanien mit dem neuen Testantigen.

Die Leishmaniose wird durch parasitäre Protozoen aus der Gattung (Genus) *Leishmania* verursacht. Die klinischen Symptome können sehr vielfältig sein, je nachdem welches Organ vorwiegend betroffen ist. Ohne Behandlung kann eine Leishmaniose tödlich enden, daher ist eine frühzeitige Diagnose von entscheidender Bedeutung, um eine gezielte Therapie einzuleiten. Man unterscheidet eine viszerale Form der Leishmaniose (VL, viszerale Leishmaniose, Kala-Azar, Dum-Dum-Fieber, Schwarzes Fieber), eine kutane Form (CL, Hautleishmaniose, Bagdadbeule, Orientbeule, Aleppobeule) und eine mukokutane Form (MCL, Schleimhautleishmaniose, Uta, Espundia). Die WHO (World Health Organisation) hat die viszerale Leishmaniose (VL) zu einer der am meisten vernachlässigten Erkrankungen erklärt und fördert daher die Diagnostik und Therapie in besonderer Weise. Neben Menschen können Leishmanien auch Tiere infizieren, vor allem Hunde, aber auch Katzen, Nagetiere, Rinder, Pferde und Meerschweinchen.

Es ist eine Vielzahl von verschiedenen Leishmanien-Stämme bekannt, beispielsweise *L. chagasi, L. donovani, L. infantum, L. major, L. braziliensis, L. archibaldi* und *L. tropica.* Die einzelnen Leishmanien-Stämme bevorzugen verschiedene Wirte und verursachen nach einer Infektion unterschiedliche klinische Symptome.

Kinesine sind eine Superfamilie von Motorproteinen, die in allen Eukaryoten vorkommen. Bei Leishmanien spielen sie eine wichtige Rolle bei der Regulation der Länge der Geißeln (Flagellum), bei der Zellteilung, dem intrazellulären Transport verschiedener Proteine und Bildung von Zytoskelettfilamenten. Das Kinesin ist bezüglich der Nukleotid - und damit auch der Aminosäuresequenz hoch konserviert und zeigt eine Identität von 80 bis 90% zwischen verschiedenen LeishmanienStämmen. Das K39-Kinesin, auch als LcKin bekannt, ist ein *Leishmania-Kinesin* und wurde ursprünglich durch Screening einer Expressionsbibliothek von *Leishmania infantum* (synonym *Leishmania chagasi)* mit Seren von Patienten mit viszeraler Leishmaniose identifiziert.

Solche Patienten zeigen eine starke Antikörperreaktion auf das Kinesin, das aus einer repetitiven Wiederholung von 39 Aminosäuren besteht. Daher sind Kinesine als diagnostisches Antigen (Testantigen für serologische Tests) geeignet. Leishmanien werden überwiegend durch Sandmücken (*Phlebotominae*) aus der Familie der Schmetterlingsmücken (*Psychodidae*) übertragen. Durch die zunehmende globale Erwärmung und die Globalisierung breiten sich die Sandmücken vornehmlich in Richtung Norden aus, wodurch die Gefahr einer Ansteckung mit Leishmanien auch in bisher wenig oder nicht betroffenen Ländern steigt. Durch intensiven Kontakt mit infizierten Haustieren, vor allem Hunden, steigt das Risiko der Leishmaniose-Infektion auch beim Menschen. In diesem Fall ist die Leishmaniose eine Zoonose.

Da die Leishmaniose vorwiegend in tropischen und subtropischen Ländern auftritt und bei Hunden auch in den Mittelmeerländern, ist die Distribution, die Lagerung und die Anwendung von Testsystemen zum Nachweis von Antikörper gegen Leishmanien eine große Herausforderung. Es gibt derzeit kein ausreichend zuverlässiges Testverfahren, Testsystem oder Testkit, mit dem mobiles ärztliches Testpersonal eine verlässliche Leishmaniendiagnostik in den verschiedenen Endemiegebieten, die durch ein unterschiedliches Erregervorkommen gekennzeichnet sind, durchführen kann. Daher bleiben viele Patienten undiagnostiziert und damit auch unbehandelt, zumal die klinischen Symptome einer Leishmaniose oftmals sehr ähnlich sind den Symptomen anderer tropischer Erkrankungen wie z.B. Malaria oder Schistosomiasis (Bilharziose).

### Stand der Technik

Um eine Leishmaniose zu diagnostizieren, bestehen zwei grundsätzliche Möglichkeiten: Entweder werden die Erreger (also die Leishmanien) direkt oder Teile des Erregers (beispielsweise dessen DNA oder bestimmte Oberflächenstrukturen) in einem Patienten nachgewiesen oder es werden Antikörper gegen Leishmanien nachgewiesen, die der Patient in Folge einer Infektion mit Leishmanien gebildet hat. Im ersten Fall wird vom Nachweis des Erregers bzw. von Bestandteilen des Erregers auf die Erkrankung geschlossen. Im zweiten Fall wird vom Antikörper-Nachweis auf die Erkrankung geschlossen.

Zum direkten Nachweis des Erregers bzw. von Bestandteilen des Erregers gehören der mikroskopische Erregernachweis, die PCR und der Antigennachweis. Der Antigennachweis, ebenso wie der Nachweis von Antikörper, beruhen auf der spezifischen Bindung von Antikörpern (diese hat der Patient gegen Leishmanien gebildet) an das Antigen (stammt von Leishmanien oder ist von Leishmanien abgeleitet worden). Diese Bindung wird anschließend durch geeignete Verfahren sichtbar gemacht. Die Verlässlichkeit des Antigen- oder Antikörpernachweises hängt ganz wesentlich von der Qualität des eingesetzten Testantigens und der Antikörper ab. Da die nachzuweisenden Antikörper üblicherweise im Blutserum eines Patienten detektiert werden, wird dieses Testverfahren als serologischer Test (Immunassay, Immunosorbent Assay, ISA) bzw. serologische Diagnostik bezeichnet. Beispiele für solche serologischen Tests sind der ELISA, der Radioimmunassay, der enzymatische Immunadsorptionstest (EIA) oder der Immunblot.

Für die Diagnostik einer Leishmaniose stehen derzeit folgende direkte und serologische Verfahren zur Verfügung:
- Direkter Erregernachweis in einer biologischen Probe (Gewebeprobe , Bioptat, Tupfpräparat, Abklatschpräparat, Aspirat, Blutausstrich, Buffy Coat-Präparat): Leishmanien können in ihrer amastigoten Form (als Leishman-Donovan-Körperchen) in weißen Blutzellen, in Knochenmarkzellen, in Milzzellen, in Lymphknotenzellen, in Leberzellen oder in Hautzellen nach Färbung mit einem geeigneten Farbstoff (beispielsweise Giemsa-Färbung, Wright- oder Leishman-Farbstoff) durch eine mikroskopische Untersuchung nachgewiesen werden. Dazu muss ausgebildetes medizinisches Personal in einem Krankenhaus oder einer spezialisierten Arztpraxis die betreffenden Gewebeproben dem Patienten entnehmen. Dieser medizinische Eingriff kann für den Patienten unangenehm, schmerzhaft oder sogar gesundheitsgefährdend sein. Für die Gewebeprobenentnahme ist unter Umständen ein Ultraschallgerät nötig, um die richtige Stelle für die Biopsie zu finden. Anschließend muss die Gewebeprobe aufbereitet und gefärbt werden. Dazu sind eine geeignete Laborausstattung und geschultes Fachpersonal notwendig. Zuletzt muss die Gewebeprobe mikroskopisch untersucht werden. Um dabei ein verlässliches Ergebnis zu erhalten, benötigt man neben viel Erfahrung auch ein geeignetes Mikroskop. Wenn die nachzuweisenden Leishmanien sich in schlecht oder zu stark gefärbten Arealen der Gewebeprobe befinden, oder von anderen Strukturen überlagert werden, dann ist ein Nachweis kaum noch möglich. Der direkte mikroskopische Erregernachweis weist also folgende Nachteile auf: Eine potentielle Schädigung des Patienten bei der Gewinnung der Gewebeprobe, ein hoher Technik- und Arbeitsaufwand, die Notwendigkeit einer vollständigen Laborausstattung und eine große Abhängigkeit von der Erfahrung des Untersuchers. Dadurch kommen subjektive Fehler mit daraus resultierenden falsch negativen Ergebnissen häufig vor. Der direkte Erregernachweis ist zusätzlich vergleichsweise teuer, zeitaufwendig und weist nur eine eingeschränkte Aussagekraft auf. Zusätzlich ist ein negatives Resultat der Untersuchung wenig aussagekräftig, da bei der Entnahme der Gewebeprobe zufällig eine Stelle getroffen werden kann, in der sich keine Leishmanien befinden und die vorhandenen Leishmanien in einer Gewebeprobe übersehen werden können.
- Erregernachweis mittels PCR (PCR-basierter Assay): Die Polymerase-Kettenreaktion (PCR) ist ein etabliertes Verfahren, um die DNA von Leishmanien in einer biologischen Probe eines Patienten nachzuweisen. Es wird also nicht der Erreger selber nachgewiesen, sondern dessen DNA, nachdem diese im Labor vervielfältigt wurde. Die Leishmaniose-Diagnostik mittels PCR weist folgende Nachteile auf: Sie dauert verhältnismäßig lange, benötigt spezifische Reagenzien, die ständig gekühlt werden müssen, ist verhältnismäßig teuer und benötigt eine aufwendige Laborausstattung und Personal mit einer großen PCR-Erfahrung. Damit ist eine PCR im Feldeinsatz nicht durchführbar. Ein weiterer, PCR-spezifischer Nachteil ist, dass es bei den kleinsten Fehlern in der Durchführung sehr leicht zu falsch positiven Ergebnissen kommen kann, beispielsweise bei einer Laborkontamination. Aber auch falsch negative Ergebnisse sind bei einer PCR häufig, wenn beispielsweise die Menge der vorhandenen DNA zu gering ist oder wenn in der biologischen Probe Hemmstoffe gegen DNA vorhanden sind.
- Nachweis von Leishmanien-Antigenen in einer biologischen Probe (Gewebe) mittels serologischer Testverfahren: Wenn ein Patient mit Leishmanien infiziert ist, dann können Bestandteile der Leishmanien wie z.B. Oberflächenstrukturen mit geeigneten Test-Antikörpern gebunden werden. Diese Bindung wird anschließend mit geeigneten Verfahren sichtbar gemacht, beispielsweise durch einen Sekundärantikörper. Dafür muss vorher dem Patienten eine geeignete biologische Probe (z.B. eine Gewebeprobe) entnommen werden, analog wie beim direkten mikroskopischen Erregernachweis. Dieses Verfahren hat folgende Nachteile: Eine potentielle Schädigung des Patienten bei der Gewinnung der Gewebeprobe und man benötigt einen oder mehrere Leishmanienstamm-spezifische Testantikörper. Zusätzlich ist ein negatives Resultat der Untersuchung wenig aussagekräftig, da bei der Entnahme der Gewebeprobe zufällig eine Stelle getroffen werden kann, in der sich keine Leishmanienantigene befinden. Die Sensitivität dieses Verfahrens ist bei Patienten mit einer klinischen Verdachtsdiagnose gering. Im Stand der Technik gibt es einen kommerziell erhältlichen Latex-Agglutinationstest (KAtex Test von Kalon Biological Ltd.), mit dem 5-20 kDa große Glykoproteine im Urin von Patienten nachgewiesen werden. Die Sensitivität dieses Tests ist gering, vor allem beim Nachweis von Leishmanien in Indien und Ostafrika. Die Spezifität dieses Tests ist bei immunkompetenten Personen gering, es kommt zu einer Vielzahl von falsch positiven Ergebnissen.
- ELISA (Enzyme-linked Immunosorbent Assay): Der ELISA ist ein enzymatisches Immunadsorptionsverfahren (EIA), mit dem Proteine oder Polypeptide (z.B. Antikörper oder Antigene) nachgewiesen werden können. Er beruht ebenfalls auf der spezifischen Bindung zwischen einem Antigen und dem korrespondierenden Antikörper. Der Antikörpernachweis mittels ELISA weist folgende Nachteile auf: Er dauert verhältnismäßig lange, benötigt spezifische Reagenzien, ist damit verhältnismäßig teuer und benötigt sowohl eine aufwendige Laborausstattung als auch geschultes Personal. Ein weiterer Nachteil des ELISAs ist die Bestimmung des richtigen Cut-Off-Punktes, mit dem ein negatives von einem positiven Signal unterschieden wird. Das ist besonders relevant bei Patienten mit einem niedrigen Leishmanien-Antikörpertiter (z.B. auf Grund einer Immundefizienz als Folge einer Infektion mit dem Humanen Immundefizienz-Virus (HIV) oder bei sonstigen Ko-Infektionen.
- RIA (Radioimmunassay): Bei einem RIA wird die spezifische Bindung zwischen einem Antigen und dem korrespondierenden Antikörper nachgewiesen, indem das Antigen mit einer radioaktiven Substanz gekoppelt wird. Über eine Messung der Radioaktivität wird auf das Vorhandensein bzw. die Menge des Antikörpers in der Probe zurückgeschlossen. Der Erregernachweis mittels RIA weist folgende Nachteile auf: Er dauert verhältnismäßig lange, benötigt spezifische Reagenzien, ist damit verhältnismäßig teuer und benötigt sowohl eine sehr aufwendige Laborausstattung als auch spezifisch geschultes Personal. Insbesondere sind radioaktive Substanzen nötig, deren Herstellung, Lagerung und Entsorgung sehr problematisch sind. Ein spezifischer Nachteil aller nuklearmedizinischen In-vitro-Testverfahren ist das Auftreten von zu niedrigen Signalen trotz hoher Konzentration des Antigens (High-Dose-Hook-Effekt).
- Immunblot (Immunoblot, IB): Der Immunblot ist ein Immunassay-Verfahren mit einer räumlich getrennten Fixierung der vorgegebenen Antikörper. Beispiele für einen Immunblot sind der Western Blot, der Dot Blot oder der Slot Blot. Das Testprinzip eines Immunblots wird auch für Schnelltests (POCT) angewandt, z.B. als Lateral-Flow-Test (LFA). Ein Immunblot in seiner ursprünglichen Form hat folgende Nachteile: Er ist sehr aufwändig und zeitintensiv und bedarf sowohl einer sehr aufwendigen Laborausstattung als auch spezifisch geschultem Personal. Damit sind auch die Kosten für einen Immunblot sehr hoch.

- Direct agglutination test (DAT): Der DAT ist ein immunchromatografisches Verfahren, welches nur in gut ausgestatteten Laboren durchgeführt werden kann und ca. 10 Stunden lang dauert. Der DAT weist folgende Nachteile auf: Er dauert verhältnismäßig lange, benötigt spezifische Reagenzien, ist damit verhältnismäßig teuer und benötigt sowohl eine sehr aufwendige Laborausstattung als auch spezifisch geschultes Personal. Das für den DAT benötigte Testantigen muss bei 4-8 °C gelagert werden, was in tropischen und subtropischen Ländern mit mangelhafter Infrastruktur nicht gewährleistet werden kann.
- Antigen-coated Dip-Stick Test: Der Dip-Stick-Test ist ebenfalls ein immunchromatografisches Verfahren, das als Schnelltest erhältlich ist. Als Probenmaterial kann Blut bzw. Blutserum verwendet werden. Der Dip-Stick-Test kann auf einem Teststreifen durchgeführt werden, der mit einem Leishmanien-spezifischen Testantigen beschichtet ist. Sind in dem Probenmaterial Antikörper gegen Leishmanien enthalten, dann binden sie an das Testantigen. Es gibt im Stand der Technik kommerziell erhältliche immunchromatografische Streifentests (ICT), die das bekannte Testantigen rK39 aufweisen (Kalazar Detect ^{™} von InBios International, Inc. sowie Onsite Leishmania Ab Rapid Test von CTK Biotech, Inc., beide als Dipstick-Test). rK39 ist ein rekombinantes Kinesinantigen aus *Leishmania infantum,* das 6,4 repetitive Regionen mit jeweils 39 Aminosäuren enthält (Repeats). Das rK39-Testantigen hat den Nachteil, dass es abhängig vom Leishmanienstamm und der Herkunftsregion eine deutlich unterschiedliche Sensitivität und Spezifität aufweist. So ist die Sensitivität und Spezifität von rK39 bei Patienten in Ostafrika sehr gering. Damit beschränkt sich der zuverlässige Einsatz von rK39-basierten Testsystemen nur auf gewisse Länder.

Bei allen serologischen Verfahren ist die Qualität des Testantigens für den Nachweis von Antikörpern entscheidend: Die Spezifität und Sensitivität eines serologischen Testes hängen von der spezifischen Bindung eines Antikörpers an das korrespondierende Antigen ab, d.h. je schlechter die Passgenauigkeit zwischen einem Antigen und Antikörper ist, desto schlechter sind die Sensitivität und Spezifität des durchgeführten Tests. Ein grundsätzliches Problem bei serologischen Tests ist das Auftreten von Kreuzreaktionen. Dabei entstehen falsch positive Ergebnisse, wenn das eingesetzte Testantigen bzw. der eingesetzte Testantikörper unspezifisch reagieren.

Kommerziell verfügbare Tests zur serologischen Diagnostik einer Leishmaniose basieren entweder auf einem Antigen von *Leishmania infantum,* das Kinesin-Protein rK39 oder einem Antigen von *Leishmania donovani, das* Kinesin-Protein rKE16. Das Testantigen rK39 ist das derzeit am meisten verwendete rekombinante Antigen für die serologische Diagnose der Leishmaniose in den Ländern Ostafrikas. In Indien wird vor allem das Testantigen rKE16 verwendet, da es hier dem rK39 überlegen ist. Da in Ostafrika die Inzidenz der viszeralen Leishmaniose weltweit am höchsten ist, zugleich aber gerade hier die Serodiagnostik bisher nur sehr unzuverlässig ist, bedarf es dringend eines vom Endemiegebiet unabhängigen, universell einsetzbaren Testantigens mit höchster diagnostischer Leistung (Sensitivität und Spezifizität).

### Aufgabe der Erfindung

Die Aufgabe der Erfindung ist es daher, ein verbessertes Verfahren für den Spezies-(Mensch/Hund) und Leishmanienstamm-übergreifenden Nachweis von Leishmanien-spezifischen Antikörpern zur Verfügung zu stellen. Das dafür notwendige Testantigen soll eine sehr hohe Sensitivität und Spezifität bei allen Stämmen zeigen, die viszerale Leishmaniose verursachen, d.h. das Verfahren soll unabhängig vom Endemiegebiet einsetzbar sein. Ebenso soll das dafür notwendige Testantigen sowohl Antikörper gegen Leishmanien in menschlichen (humanen) biologischen Proben (z.B. in Serum), als auch in biologischen Proben von Hunden (canines Serum) nachweisen. Das verbesserte Verfahren soll für den weltweiten Einsatz zum Nachweis von Antikörpern gegen Leishmanien bei Menschen und Hunden geeignet sein. Für dieses Verfahren wird ein geeigneter Test beschrieben. Ebenso wird ein Verfahren zur Herstellung dieses neuen Testantigens beschrieben. Das Testantigen soll keine Kreuzreaktivität mit Malaria, Tuberkulose und anderen Koinfektionen aufweisen. Ebenso soll das Testantigen stabil über einen längeren Zeitraum (mehrere Jahre) lagerbar sein, ohne seine biologischen Funktionen zu verlieren.

### Lösung der Aufgabe

Die vorliegende Erfindung löst die Aufgabe durch die Bereitstellung des neuen, künstlich hergestellten Testantigens rKLi8.3, das eine immunologisch aktive Aminosäuresequenz gemäß SEQ ID NO. 8 aufweist und in dem erfindungsgemäßen Verfahren eingesetzt wird. Die SEQ ID NO. 7 ist die Nukleinsäuresequenz des erfindungsgemäßen Testantigens, die SEQ ID NO. 8 ist die dazu korrespondierende Aminosäuresequenz. Die Abkürzungen für die Nukleinsäuren und die Aminosäuren sind dem Fachmann bekannt. Dieses künstlich hergestellte Testantigen reagiert mit Antikörpern gegen verschiedene Leishmanienstämme, sowohl bei Menschen als auch bei Hunden. Die Bindung des neuen, künstlich hergestellten Testantigens mit Antikörpern von Leishmanien-infizierten Menschen oder Hunden weist eine sehr hohe Spezifität und Sensitivität auf. Dies wird in wissenschaftlichen Untersuchungen der Erfinder gezeigt. Die Antikörper-Testantigen-Bindung des neuen, künstlich hergestellten Testantigens mit den nachzuweisenden Antikörpern von Menschen oder Hunden wird mit einer der bekannten Methoden unter Verwendung eines bekannten Standardverfahrens sichtbar gemacht, beispielsweise mit einer Farbreaktion (chromogene, kolorimetrische Verfahren, Chemilumineszenz, Fluoreszenz oder mittels Goldkolloid). Im positiven Fall, also bei einem Nachweis von Antikörpern, wird auf eine Infektion des zu testenden Patienten (Mensch/Hund) mit Leishmanien zurück geschlossen.

Ebenso umfasst die vorliegende Erfindung einen Test (Testsystem, Testkit, Schnelltest) unter Verwendung des neuen, künstlich hergestellten Testantigens.

Die derzeit verfügbaren serologischen Tests zeigen jedoch in verschiedenen Endemiegebieten eine unterschiedliche diagnostische Sensitivität und Spezifität, weshalb mit einem Testantigen Infektionen mit Leishmanien oft nicht zuverlässig diagnostiziert werden kann. Grund hierfür kann die Stamm-spezifische Sequenz (Kinesinsequenzen variieren in unterschiedlichen Leishmanienstämmen), die Struktur des Kinesin-Testantigens (Anzahl der Kinesinrepeats), bzw. die Kombination von beiden sein. Diese Aspekte haben die Erfinder systematisch analysiert. Das Ergebnis ihrer Studien ist, dass neben der Sequenzvariabilität die Kinesinantigenstruktur (Anzahl der Repeats) einen dominanten Einfluss auf die diagnostische Sensitivität und Spezifität hat.

Hierzu wurden Kinesinproteine mit einer zunehmenden Anzahl von Repeats aus verschiedenen Leishmanienstämmen rekombinant hergestellt und auf die Bindungsstärke von Antikörpern aus Patienten mit viszeraler Leishmaniose (VL) getestet. Dabei zeigte sich, dass die Antikörperbindung an das Kinesin-Testantigen stärker ist, wenn die Zahl der Repeats zunimmt. Somit konnten die Erfinder zeigen, dass die Anzahl der Repeats die Affinität zwischen Testantigen und Antikörper erhöht. Darauf wurden rekombinante Kinesinproteine aus verschiedenen Leishmanienstämmen mit unterschiedlicher Sequenz aber gleicher Repeatanzahl analysiert. Es zeigte sich, dass neben der Repeatanzahl auch die Aminosäuresequenz der Kinesine einen Einfluss auf die Antikörperbindung hat. Hier zeigte sich, dass stark konservierte Kinesinproteine, die hydrophile Eigenschaften aufweisen, am besten mit den Antikörpern der Patienten reagieren und somit am besten als Testantigene geeignet sind.

Wenn Testantigene kloniert werden, dann sind sich wiederholende DNA-Sequenzen (tandem repeats), wie hier das Kinesin, meist nur sehr schwer zu klonieren und zu exprimieren. Die Arbeit mit repetitiver DNA ist mit vielen Herausforderungen verbunden. Das Amplifizieren von mehreren tandem repeats mittels PCR ist schwierig, da vorzugsweise kürzere Fragmente amplifiziert werden und lange Sequenzbereiche sehr ineffizient transkribiert und translatiert werden.

Außerdem können durch die PCR-Amplifikation vermehrt Rekombination oder Chimärenbildung entstehen. Molekulare Mechanismen, die zur Erzeugung solcher Rekombination führen, sind meist unbekannt oder unklar. In Bakterien werden repetitive DNA-Sequenzen häufig rekombiniert oder eliminiert. Zudem treten in Bakterien, die mit repetitiven Sequenzen transformierten wurden, häufig toxische Effekte auf, weshalb das gewünschte Protein nicht oder nur unvollständig exprimiert wird.

Da für das erfindungsgemäße Verfahren ein neues Testantigen entwickelt werden musste, befassten sich die Erfinder als erstes mit der Entwicklung eines neuen verbesserten Testantigens.

Die vorliegende Erfindung umfasst auch die Herstellung des neuen Testantigens aus selektionierten Leishmanienstämmen mittels PCR und Klonierung in kompetenten E. coli-Bakterien. Folgende Schritte werden dafür durchgeführt:
(i) Bereitstellen von promastigoten Leishmanien;
(ii) Isolieren der genomischen DNA aus den promastigoten Leishmanien aus Schritt (i);
(iii) Amplifizieren des Genabschnitt aus der DNA aus Schritt (ii) gemäß SEQ ID NO. 3 mit dem Forward-PCR-Primer gemäß der SEQ ID NO. 1 und dem Reverse-PCR-Primer gemäß der SEQ ID NO. 2, so dass ein Amplifikationsprodukt entsteht;
(iv) Isolation und Aufreinigung des Amplifikationsproduktes aus Schritt (iii);
(v) Klonierung des aufgereinigten Amplifikationsproduktes aus Schritt (iv) in einen Vektor;
(vi) Transfektion von kompetenten E. *coli* mit dem Vektor aus Schritt (v);
(vii) Expression des Testantigens in E. *coli*;
(viii) Lyse der E. *coli* aus Schritt (vii);
(ix) Isolation des Testantigens aus den lysierten E. *coli aus* Schritt (viii);
(x) Aufreinigung des Testantigens aus Schritt (ix).

Im Einzelnen werden die Herstellungsschritte genauer beschrieben:
- Kultivierung von promastigoten Leishmanien im Zellkulturmedium RPMI-1640 mit L-Glutamin, NaHCO₃ und 10% (v/v) FCS (Fetal Calf Serum)
- Isolierung der genomischen DNA aus kultivierten promastigoten Leishmanien
- Herstellung der Forward- und Reverse-PCR-Primer: Der Forward-Primer hat die SEQUENZ ID NO. 1 und der Reverse-Primer hat die SEQUENZ ID NO. 2. Der amplifizierte Genabschnitt hat die SEQUENZ ID NO. 3. Das Kinesin-Antigen liegt auf dem Chromosom 14 von *Leishmania.* Dieses Antigen ist ein Teil des KinesinProteins von *Leishmania.* Im Rahmen der erfinderischen Tätigkeit wurden zur Identifizierung eines universell und ubiquitär (unabhängig vom Leishmanienstamm und Endemiegebiet) verwendbaren Kinsesin-Testantigens hochkonservierte Kinesinsequenzen aus 7 ostafrikanischen *Leishmania*-Isolaten durch Kultivierung, Klonierung und Sequenzierung der Leishmanien ermittelt und mit bisher publizierten Kinesinsequenzen verglichen. Diese Analysen führten zur rationalen Selektion (Identifikation) eines optimalen (stammesunabhängigen, d.h. Leishmanienisolat-unabhängigen) Antigens, das aus der Amplifikation von 8.3 Repeats bei *L. infantum* aus Ostafrika entsteht.

Die weitere erfinderische Tätigkeit nach der Identifikation des stammesunabhängigen Testantigens beinhaltet folgende Methodik:
- Durchführung der PCR-Reaktion
- Isolation des Amplifikationsproduktes von rKLi8.3 mit 8,3 Repeats und 1117 Basenpaaren aus dem Gel
- Klonierung des gereinigten Amplifikationsproduktes in einen Vektor, beispielsweise dem pCR^{®}2.1-TOPO Vektor (Invitrogen).
- Transformation von kompetenten E. *coli* mit dem Vektor aus dem vorhergehenden Schritt.
- Expression des rekombinanten Testantigens rKLi8.3 in E. *coli*
- Zerstörung (Aufschluss, Lyse) der E. *coli,* beispielsweise mittels eines Mikrofluidizers, um das rekombinante Testantigen rKLi8.3 freizusetzen.
- Aufreinigung des in E. *coli* exprimierten rekombinanten Testantigens rKLi8.3 aus dem Bakterienlysat aus dem vorhergehenden Schritt beispielsweise mittels Ni-Affinitätschromatografie und anschließender Größenausschluss-Chromatografie.

Um das Testantigen für das erfinderische Verfahren herzustellen, mussten folgende Schwierigkeiten beim Klonieren der hochrepetitiven Sequenzen überwunden werden:
- Die DNA der Kinesin-Gene weist einen sehr hohen GC-Anteil (>65%) auf. Ein hoher GC-Anteil verändert die Sekundärstruktur und erhöht die Stapelwechselwirkungen, weshalb die Amplifikation solcher Fragmente durch die Polymerase-Kettenreaktion (PCR) häufig zu unerwünschten Produkten führt.

Daher haben die Erfinder systematisch verschiedene Aspekte untersucht, wie solche unerwünschten Rekombinationen entstehen und wie sie beseitigt werden können. Diese Probleme wurden folgendermaßen gelöst:
- Optimierung des PCR-Programms
- Verwendung unterschiedlicher Annealing-Zeiten
- Verwendung unterschiedlicher Annealing-Temperaturen
- Verwendung verschiedener Denaturierungstemperaturen
- Verwendung von Primern mit unterschiedlichen Annealing-Stellen
- Verwendung verschiedener DNA-Konzentrationen
- Verwendung von kompetenten recA knockout E. *coli*: RecA ist ein 38 Kilodalton großes Protein in E. *coli,* welches bei der Reparatur und Erhaltung von DNA eine Rolle spielt. RecA (E. *coli*) besitzt ein Reparatursystem, das homologe Sequenzen rekombiniert. Die Amplifikation repetitiver eukaryontischer DNA-Sequenzen in Bakterien ist aufgrund von Umlagerung oder Deletion durch DNA-Reparatursysteme (recA) in E. *coli* problematisch. Diese Umlagerungen führen zu folgenden nachteiligen Effekten:
- Deletion oder Duplikation von Genen, die von direkten Sequenzwiederholungen flankiert sind
- Änderung der Anzahl sich wiederholender Elemente
- Rekombinationsereignisse

Um diese negativen Effekte zu überwinden, wurden spezielle Bakterienstämme verwendet, die zum Klonieren der instabilen, repetitiven DNA geeignet sind. Diese Stämme tragen Mutation in ihren Rekombinasen-Genen (z.B. recA1, recB, recA13). recA13 und recA1 können die Rekombination klonierter DNA reduzieren und sind deshalb für das Klonieren von einer größeren Anzahl von tandem repeats geeignet. Es konnte ermittelt werden, dass eine hochrepetitive DNA eine hohe Denaturierungstemperatur benötigt, damit der Doppelstrang mit Tandemsequenzen effizient geöffnet wird und die Polymerase gut andocken kann.

Ebenso wurde ermittelt, dass das Binden des Primers an die DNA (Annealen) nicht notwendigerweise am 3'-Ende beginnt, sondern aufgrund der repetitiven Struktur (Repeats) über die gesamte Länge des Strangs erfolgen kann. Daher konnte durch das Verlängern des nicht-repetitiven Prä-Repeats ein Position-definiertes Annealen der Primer erreicht werden.

Um das erfindungsgemäße Verfahren durchzuführen, muss vorher eine biologische Probe des zu testenden Patienten (Menschen oder Hunde) zur Verfügung gestellt werden. Die biologische Probe ist in der Regel Serum (Blutserum), sie kann aber auch Vollblut, Plasma (Blutplasma), Lymphknotenaspirate, Speichel, Gewebsflüssigkeit, Liquor cerebrospinalis, Urin oder eine andere körpereigene Flüssigkeit sein, welche im Fall einer Infektion mit Leishmanien Antikörper gegen diese enthält. Das erfindungsgemäße Verfahren zum Nachweis von Antikörper gegen Leishmanien umfasst folgende Schritte:
(i) Bereitstellung einer biologischen Probe eines Menschen oder eines Hundes;
(ii) Bereitstellung des auf einer Unterlage gebundenen Testantigens mit der SEQ ID NO. 8, das 8,3 repetitive Sequenzen aufweist und das von Antikörpern gegen verschiedene Leishmanienstämme erkannt und gebunden werden kann;
(iii) Inkubation der biologischen Probe aus Schritt (i) mit dem Testantigen aus Schritt (ii), so dass Antigen-Antikörper-Komplexe gebildet werden, wenn in der biologischen Probe aus Schritt (i) Antikörper gegen mindestens einen Leishmanienstamm enthalten sind;
(iv) Entfernung von nicht an das Testantigen aus Schritt (ii) gebundene Antikörper und sonstigem Material aus der biologischen Probe aus Schritt (i) in einem ersten Waschschritt;
(v) Zugabe eines human-spezifischen Sekundärantikörpers, wenn die biologische Probe aus Schritt (i) von einem Mensch stammt oder Zugabe eines canin-spezifischen Sekundärantikörpers, wenn die biologische Probe aus Schritt (i) von einem Hund stammt, wobei der jeweilige Sekundärantikörper durch ein Mittel zur Sichtbarmachung einer Bindung des Sekundärantikörpers nachweisbar ist, so dass der jeweilige Sekundärantikörper an die Antigen-Antikörper-Komplexe aus Schritt (iii) bindet;
(vi) Entfernung von nicht gebundenen Sekundärantikörpern aus Schritt (v) in einem zweiten Waschschritt;
(vii) Sichtbarmachung der Bindung des Sekundärantikörpers an den Antigen-Antikörper-Komplexen aus Schritt (iii) durch Zugabe eines geeigneten Mittels.

Die Bindung der nachzuweisenden Antikörper an das Testantigen und die Sichtbarmachung dieser Antikörper-Testantigen-Bindung kann mit bekannten Methoden durchgeführt werden, beispielsweise mittels ELISA, immunchromatografischer Verfahren oder mittels eines Schnelltests (Dipstick, Line-Blot). Dem Fachmann sind die dazu benötigten Materialien und Verfahren bekannt.

In einem bevorzugten Ausführungsbeispiel wird Antikörper-Testantigenbindung mit einem anti-humanen Sekundärantikörper nachgewiesen. Ein Beispiel dafür ist der Peroxidase-konjugierte Donkey anti-human IgG (H+L) von Jackson Immunoresearch Laboratories, USA.

In einem anderen bevorzugten Ausführungsbeispiel wird Antikörper-Testantigenbindung mit einem anti-caninen Sekundärantikörper nachgewiesen. Ein Beispiel dafür ist der Peroxidase-konjugierte Rabbit anti-Dog IgG (H+L) von Jackson Immunoresearch Laboratories, USA.

In einem anderen Ausführungsbeispiel kann die Antikörper-Testantigenbindung mit jedem Sekundärantikörper nachgewiesen werden, der gegen humanes IgM/IgG gerichtet ist.

In einem weiteren Ausführungsbeispiel kann die Antikörper-Testantigenbindung mit jedem Sekundärantikörper nachgewiesen werden, der gegen canines IgM/IgG gerichtet ist.

In einem Ausführungsbeispiel wird das erfindungsgemäße Verfahren als Dip-Stick-Test (Streifentest, Schnelltest, ICT) umgesetzt. Bei diesem immunchromatografischen Verfahren kann als Probenmaterial Blut bzw. Blutserum verwendet werden. Der Dip-Stick-Test wird auf einem Teststreifen durchgeführt, welcher mit dem erfindungsgemäßen Testantigen beschichtet ist. Sind in der biologischen Probe Antikörper gegen Leishmanien enthalten, dann binden sie am erfindungsgemäßen Testantigen. Diese Antikörper-Testantigenbindung wird durch ein dem Fachmann bekanntes immunchromatografisches Verfahren sichtbar gemacht.

Wird das erfindungsgemäße Verfahren nicht als ELISA durchgeführt, sondern als Dip-Stick-Test, Lineblot oder lateral diffusion test, dann muss das erfindungsgemäße Verfahren in einer dem Fachmann bekannten Weise modifiziert werden, beispielsweise durch den Wegfall der Waschschritte oder der Mitführung einer Negativkontrolle.

### Ausführungsbeispiele

Die Ausführungsbeispiele erläutern die erfinderische Tätigkeit der Erfinder, welche zu dem neuen Testantigen geführt hat.

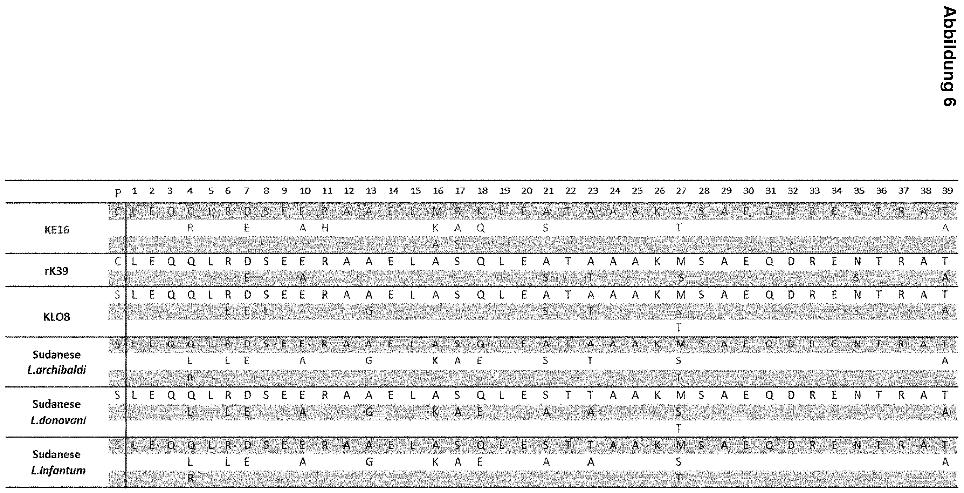

Ausgehend von der beschränkten Sensitivität und Spezifität der bekannten Testantigene haben die Erfinder nach einem verbesserten Testantigen geforscht. Mit Hilfe verschiedener Leishmanienisolate überprüften sie den Einfluss der Antigen-Struktur (Einfluss der Repeatanzahl) und -Sequenz (Einfluss von Sequenzvariationen) auf Antigenität und stammübergreifende Konservierung. Hierzu haben die Erfinder über 53 Kinesin-Fragmente verschiedener *Leishmania*-Arten und -Stämme aus dem Sudan kloniert, sequenziert und bioinformatisch analysiert. In der Abbildung 6 sind alle Aminosäurevariationen der 53 analysierten Kinesinfragmente aus 7 Isolaten (zweimal *L. archibaldi*; zweimal *L.infantum* und dreimal *L. donovani*) zusammengefasst. Zunächst wurde eine nicht-repetitive Sequenz, das Prä-Repeat von Kinesin, verglichen (46 Aminosäuren). Es zeigte sich, dass die Aminosäuresequenz der Prä-Repeats verschiedener Leishmania-Stämme hoch konserviert ist. Nur in Position 41 variiert die Sequenz zwischen den Aminosäuren Cystein und Serin. In der nicht wiederholenden 46aa-Region war die einzige Abweichung von *L. infantum* (*L. chagasi*) rK39 und dem KE16 von *L*. *donovani* Cys→Ser⁴¹.

Die Erfinder zeigen, dass ostafrikanische Sequenzen gegenüber dem rK39 mehrere Sequenzvariationen aufweisen. Die meisten Variationen innerhalb der 5'-Repeat-region sind nicht konserviert, wobei mehrere Substitutionen mit Ladungsänderungen einhergingen, während die Aminosäure-Substitutionen in der 3'-Hälfte der Repeats meistens konserviert waren. Wurde die rK39-Aminosäuresequenz mit 53 Konstrukten verglichen, die Kinesinfragmente ostafrikanischer Stämme enthielten, so waren besonders die Position 4, 6, 16 und 18 von Substitutionen betroffen, die mit Ladungsänderungen verbunden waren. Bei allen Stämmen aus dem Sudan war die Diversität in der ersten Hälfte jedes Kinesin-Repeats besonders auffallend, da sie geladene Aminosäuren an den obengenannten Positionen enthalten. Solche Ladungsänderungen können die Antigenität des Kinesinproteins verringern. Die Erfinder können vorhersagen, dass die diagnostischen Epitope besonders in der zweiten Hälfte der Kinesin-Wiederholung liegen, da dieser Abschnitt (Aminosäuren 28-34) in allen untersuchten Isolaten vollständig konserviert ist.

Folgende Polymorphismen gegenüber rK39 wurden in ostafrikanischen Isolaten identifiziert: Gln→Leu⁴, Gln→Arg⁴, Arg→Leu⁶, Ser→Leu⁸, Ala→Gly¹³, Ala→Lys¹⁶, Ser→Ala¹⁶, Gln→Glu¹⁸ und Met→Thr²⁷.

Die Erfinder haben festgestellt, dass es eine große genetische Diversität von Kinesinen zwischen *Leishmania*-Stämmen aus Ostafrika, Indien und Brasilien gibt.

Diese Heterogenität der Kinesin-Antigene erklärt, warum rK39 (Brasilien) und rKE16 (Indien) in Afrika eine geringe Leistung zeigen.

Das Genfragment, das die immundominanten Wiederholungen von *L. infantum* (strain MHOM/SD/82/GILANI) aus Sudan codiert, hier bezeichnet als KLi8.3, wurde aus genomischer DNA aus promastigoten Leishmanien amplifiziert. Die Leishmanien wurden im Medium RPMI-1640 mit L-Glutamin, NaHCO3 (Sigma-Aldrich) und 10 % (v/v) FCS (Sigma-Aldrich) kultiviert und genomische DNA wurde nach Standardprotokollen hergestellt.

Die Forward- (5'-GAGCTCGCAACCGAGTGGGAGG-3') und Reverse- (5'-GCTCCGCAGCGCGCTCC-3') PCR-Primer (SEQ ID NO. 1 und 2) wurden entsprechend dem veröffentlichten *Leishmania infantum* JPCM5 putative kinesin K39 (GenBank: XM_001464261.2) entworfen. Die PCR-Reaktion wurde mit Phusion^{®} High-Fidelity DNA-Polymerase (FINNZYMES OY, Finnland) durchgeführt. Die Reaktion wurde in insgesamt 50 µl durchgeführt, die 5 % (v/v) DMSO, 4 mM MgCl₂, 10 µl GC-Puffer, 10 mM dNTPs mix (Thermo Scientific) und 200 ng genomische DNA enthielt. Die PCR wurde wie folgt durchgeführt: Denaturierung bei 98 °C für 30 s, gefolgt von 35 Zyklen mit Denaturierung bei 98 °C für 10 s, Annealing bei 71,1 °C für 30 s und Extension bei 72 °C für 60 s. Die amplifizierten Produkte zeigten mehrere Banden mit einer Größe, die ein-bis mehrfachen 117bp-Wiederholungen entsprechen. Das größte Amplifikationsprodukt (1117bp) wurde aus dem Gel gereinigt und in einen pCR^{®}2.1-TOPO Vektor (Invitrogen) kloniert. Kompetente Zellen aus E. coli-Bakterien HB101 (Promega) wurden mit dem rekombinanten Plasmid pCR2.1/KLi8.3 transformiert. Die klonierte Sequenz wurde durch Restriktionsverdau mit *EcoRI* (NEB) und durch Sequenzanalyse bestätigt. Die Sequenz ist identisch mit SEQ ID NO. 3.

Tandemwiederholungen der Sequenz von KLi8.3 wurden mit dem Programm *Tandem Repeats Finder* lokalisiert und angezeigt (SEQ ID NO. 4).

Zur Expression des rekombinanten Testantigens wurde die für KLi8.3 kodierende DNA-Sequenz in den his-tag-Vektor pET28a (+) (EMD Biosciences) subkloniert. Als PCR DNA-Template wurde das DNA-Konstrukt pCR2.1/KLi8.3 mit dem Forward-Primer 5'-GTACATATGGAGCTCGCAACCGAGTGGGAGGACGCA-3' und dem Reverse-Primer 5'-TACCTCGAGCAGTGTGCTGGAATTCGCCCTTACTCCGCAGC-3' (SEQ ID NO. 5 und 6) verwendet. Die Amplifikation erfolgte mit Phusion Hot Start II DNA Polymerase (Thermofisher Scientific, USA) gemäß den Empfehlungen des Herstellers. Die amplifizierten DNA-Fragmente wurden mit Ndel und Xhol Restriktionsenzymen verdaut und in-frame und downstream von 6x His-tag in die entsprechenden Stellen des Vektors pET28a (+) kloniert, um das Plasmidkonstrukt pET28a/KLi8.3 zu erzeugen.

SEQ ID NO. 7 zeigt das gesamte DNA-Konstrukt, das für das his-KLi8.3-Fusionsprotein kodiert. Die SEQ ID NO. 7 setzt sich aus der SEQ ID NO. 3 mit 6 x Histidinresten zusammen, die durch folgende Nukleotide kodiert sind: CATCATCATCATCATCAC.

Mit Hilfe von 6xhis-Resten wurde das rKli8.3 Protein über Ni-A Affinitätssäule gereinigt. Das rekombinante Plasmid wurde durch DNA-Sequenzierung und Restriktionsanalyse verifiziert und anschließend in NEB 5-alpha F'lq Competent E. coli-Bakterien (New England Biolabs) transformiert.

Die Expression von rKLi8.3 in transformierten BL21(DE3) kompetenten E. *coli-*Bakterien wurde nach Zugabe von 1 mM Isopropyl-β-D-1-thiogalactopyranosid (IPTG) und Inkubation für 3 h bei 37 °C und 200 U/min induziert. Die Zellen wurden im Microfluidizer lysiert und die löslichen Fraktionen wurden durch Zentrifugation gewonnen. Das rekombinante Testantigen wurde durch Ni2+-Affinitätschromatografie mit HisTrap HP 5 mL Säule (GE Healthcare, USA) gereinigt. Mit der Größenausschlusschromatografie wurden letzte Verunreinigungen und eventuelle Aggregate entfernt. Die Chromatografie wurde mit Hilfe einer ÄKTA-Chromatografie-Anlage (GE Healthcare, USA) durchgeführt. Das gereinigte rKLi8.3-Protein mit 393 Aminosäuren und 6 Histidinresten (SEQ ID NO. 8) und einem Molekulargewicht von 43,2 kDa wurde durch SDS-PAGE aufgetrennt. Bakterielle Extrakte, die das Plasmid pET28a/rKLi8.3 enthalten, wurden auf 12,5%iger SDS-PAGE aufgetrennt und vor (0 h) und nach (3 h) Induktion mit IPTG mit Coomassie Blue gefärbt. Das rekombinante Testantigen wurde durch Ni2+-Affinitätschromatografie mit HisTrap HP 5 mL Säule (GE Healthcare, USA) gereinigt.

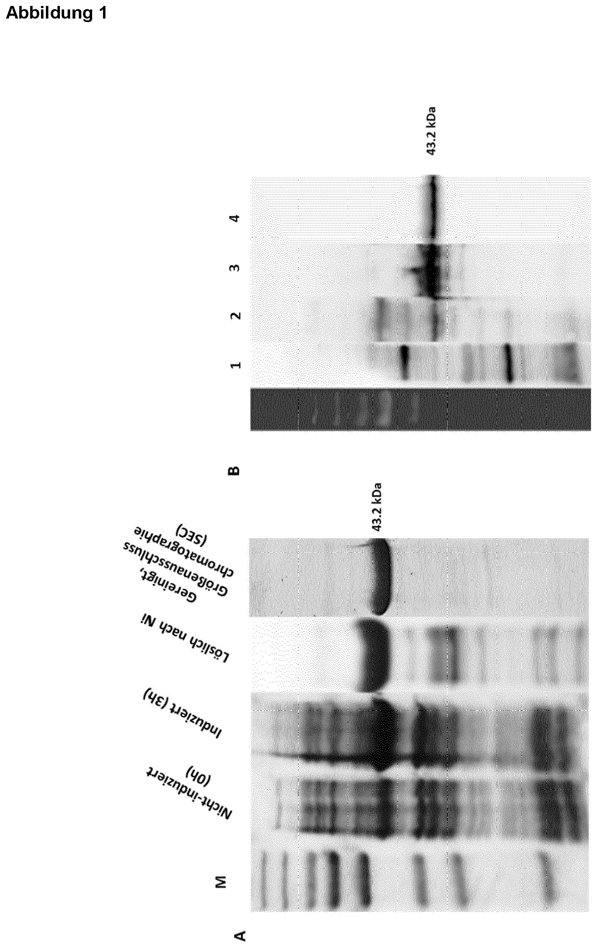

Die Reaktivität des rekombinanten Testantigens rKLi8.3 wurde im Western-Blot mit 10 gepooltem Seren von Patienten mit bestätigter *L*. *donovani*-Infektion und 10 gepoolten Kontrollseren gesunder Personen aus dem Sudan untersucht. Dazu wurden die Testantigene auf eine Nitrocellulose-Transfermembran (Whatman GmbH, Deutschland) unter Verwendung von Bio-Rad Semi-dry Trans-Blot bei 200 mA für 1 h übertragen. Die Membran wurde dann mit 5% BSA (w/v) in 100 mM NaCl, 0,05% Tween 20 (v/v) und 10 mM Tris-HCI, pH 7,4 (Blocking-Puffer) blockiert und dann 18 Stunden lang bei 4 °C mit entweder Patienten- oder Kontrollseren (1:1000 in Blocking Puffer) inkubiert. Nach dem Waschen wurden die Blots 1 Stunde lang bei einer Temperatur zwischen 18 und 25 °C (Raumtemperatur, RT) mit Peroxidase-konjugiertem Esel-Antihuman-IgG (H+L) (Jackson Immunoresearch Laboratories, USA) inkubiert (1:10000 Verdünnung). Wie in Abbildung 1 gezeigt, wurde das positive Serum von dem rekombinanten Testantigen rKLi8.3 (Spur 3 und 4) erkannt, während das negative Serum nicht mit dem rekombinanten Testantigen rKLi8.3 reagierte (Spur 1). Diese Ergebnisse bestätigten, dass rKLi8.3 als Testantigen für den spezifischen Nachweis von *Leishmania*-Antikörpern sehr gut geeignet ist.

In einem Ausführungsbeispiel wird das erfindungsgemäße Testantigen in einem ELISA zum serologischen Nachweis von Antikörpern gegen Leishmanien eingesetzt. Der ELISA wurde mit MaxiSorp^{™} Polystyrol-ELISA-Platten mit hoher Proteinbindungskapazität durchgeführt (NUNC TM Serving Life Science, Dänemark). Zunächst wurden die Proteinkonzentration für das Beschichten (Coating) der Platten zur Bestimmung der optimalen Serumverdünnungen analysiert. Hierzu wurden gepoolte Seren von 10 charakterisierten sudanesischen Patienten mit bestätigter viszeraler Leishmaniose und 10 gepoolte Kontrollseren von gesunden Personen aus dem Sudan verwendet. Verschiedene Konzentrationen des erfindungsgemäßen Testantigens wurden gegen serielle Verdünnungen positiver oder negativer Seren titriert. 5 bis 50 ng rekombinantes Testantigen rKLi8.3 pro Well wurden über Nacht bei 4 °C in 0,1 M NaCO3-Puffer pH 9,6 auf ELISA-Platten beschichtet. Die Platten wurden mit PBS mit 0,05% (v/v) Tween-20 gewaschen und dann mit 3% (w/v) BSA im gleichen Puffer bei einer Temperatur zwischen 18 und 25 °C (Raumtemperatur, RT) für 1 Stunde blockiert. Nach weiteren Waschschritten wurden 50 µl verdünnte positive oder negative Serumproben in jedes Well gegeben und die Platten 45 Minuten lang bei einer Temperatur zwischen 18 und 25 °C (Raumtemperatur, RT) inkubiert. Nach dem Waschen wurde 50 µl/Well Peroxidase-konjugiertes AffiniPure Donkey Anti-Human IgG (H+L) (Jackson Immunoresearch Laboratories, USA) (1:10000) in jedes Well gegeben und die Platten weitere 1 h bei einer Temperatur zwischen 18 und 25 °C (Raumtemperatur, RT) inkubiert. Die Reaktion wurde mit Wasserstoffperoxid und Tetramethylbenzidin (R&D Systems, USA) visualisiert. Die Reaktion wurde mit 2 M Schwefelsäure nach 10 Minuten Inkubation im Dunkeln gestoppt. Die optische Dichte (OD) wurde bei 450/570 nm mit einem ELISA-Microreader (FLUOstar Omega, BMG LABTECH) gemessen. Jede Probe wurde mindestens zweimal getestet und der Mittelwert wurde berechnet. Proben mit ungültigen oder widersprüchlichen Ergebnissen wurden wiederholt.

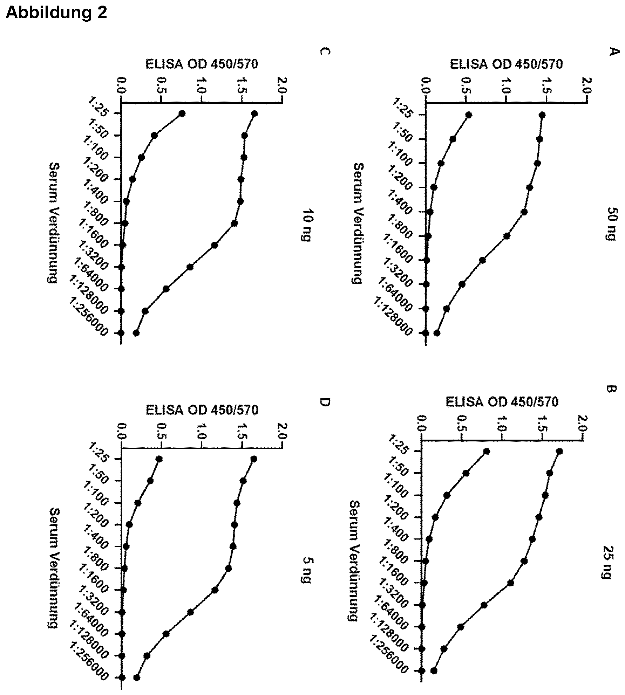

Alle getesteten Konzentrationen (5-50 ng/well) von rKLI8.3 wurden von den gepoolten Seren von Leishmaniosepatienten erkannt (s. Abbildung 2). Seren gesunder Personen reagierten nicht mit dem erfindungsgemäßen Testantigen. Die ODs positiver Seren waren mindestens 4-fach höher als bei negativen Seren. Das Beschichten (Coating) von ELISA-Platten mit einer Konzentration von 5 ng Testantigen war ausreichend für den positiven Nachweis von Seren von *Leishmania*-infizierten Patienten. Daher wurde eine Proteinkonzentration von 5 ng/Well und eine Serumverdünnung von 1:800 als optimale Bedingungen für den Nachweis ausgewählt und in den folgenden Experimenten angewandt.

Die diagnostische Leistung aller aus dem Stand der Technik bekannten rekombinanten Testantigene wurde unter Verwendung von diagnostischen Effizienzwerten bestimmt. Zur Berechnung der entsprechenden diagnostischen Parameter wurden folgende Definitionen verwendet: true-positive (tp) = Seren von Patienten mit bestätigter viszeraler *Leishmania*-Infektion; false-negative (fn) = Seren von Patienten mit bestätigter viszeraler *Leishmania*-Infektion mit negativen Werten; false-positive (fp) = Seren von gesunden Personen ohne viszerale *Leishmania*-Infektion mit positiven Werten; true-negative (tn) = Seren von gesunden Personen ohne viszerale *Leishmania*-Infektion mit negativen Werten; Positive predictive value (PPV) = Wahrscheinlichkeit, dass die Krankheit vorhanden ist, wenn der Test positiv ist, TP / (TP + FP) × 100%; Negative predictive value (NPV) = Wahrscheinlichkeit, dass die Krankheit nicht vorhanden ist, wenn der Test negativ ist, TN / (TN + FN) × 100%; Sensitivität = tp × 100/(tp + fn); Spezifizität = tn × 100/(tn + fp). Die Werte für die diagnostische Effizienz (DEV) wurden aus (tn + tp) × 100/(tp + fp + tn + fn) berechnet.

Eine Gesamtzahl von 288 positiven oder negativen Humanseren wurden für die Untersuchungen verwendet. 172 Seren stammten von Patienten mit viszeraler Leishmaniose (VL), die durch Lymphknotenaspiration bestätigt war. 85 Seren stammten von gesunden Personen, die in einem endemischen Gebiet für VL wohnten. Als weitere Kontrollen wurden Seren von sudanesischen Patienten, die an anderen häufigen Krankheiten in den endemischen Gebieten leiden, in die Studie aufgenommen. 5 Seren stammten von Patienten mit bestätigter Malaria und 26 von Patienten mit diagnostizierter Tuberkulose. Alle Seren wurden bei -20 °C gelagert. Es wurden auch 10 Kontrollseren von nicht infizierten deutschen Personen gesammelt und eingesetzt.

Der ELISA wurde wie oben beschrieben mit dem erfindungsgemäßen Testantigen rKLi8.3 durchgeführt. Die Patienten- und Kontrollseren wurden im Verhältnis 1:800 verdünnt und mit 5 ng rKLi8.3 pro Well getestet.

Die Daten wurden mit der Software GraphPad Prism (GraphPad Prism Inc., San Diego, Ca) ausgewertet. Die gesunden Kontrollen aus dem Sudan wurden zur Bestimmung des ELISA-Cut-off-Werts verwendet. Diese wurden für jedes rekombinante Testantigen als mittlerer Absorptionswert von Seren gesunder Kontrollen plus 3 × Standardabweichungen (SD) festgelegt.

Das aus dem Stand der Technik bekannte rekombinante Testantigen rk39 von *Leishmania chagasi* mit der GenBank-Zugangsnummer AAA29254.1 wurde von Rekom Biotech, S.L., Granada, Spanien, gekauft. Es wurde auch als Fusionsprotein mit einem 6× his-Tag am C-Terminus in E. *coli* exprimiert. Nach der Lieferung wurde die Konzentration des Testantigens mit der gleichen Methode überprüft, die zur Messung der rekombinanten Testantigene rKLO8 und rKLi8.3 durchgeführt wurde (Bradford). Aliquots wurden bei -80 °C gelagert. In der Tabelle 1 sind alle getesteten Seren mit ihrer Herkunft und ihren Eigenschaften aufgelistet.

**Tabelle 1: Seren von Patienten mit viszeraler Leishmaniose (VL) und Kontrollen aus dem Sudan und Deutschland. Abürzungen: VL, viszerale Leishmanoise; NEC, nicht-endemische Kontrollen; EC, endemische Kontrollen; MA, Malaria; TB, Tuberkulose; AFB, Acid Fast Bailli**

| **Herkunft (Land)** | **Nr. von Seren** | **Klinischer Fall** | | **Eigenschaften** | **Klassifizierung** |
|---|---|---|---|---|---|
| Sudan | 288 | VL | (n=172) | Die Diagnose basiert auf dem Nachweis von Parasiten in gefärbten Lymphknotenabstrichen | Bestätigte VL Patienten |
| | | EC | (n=85) | Aus dem gleichen VL-Endemiegebiet (Sudan) | Negativkontrollen |
| | | MA | (n=5) | Die Diagnose erfolgte durch den Nachweis von Malaria Parasiten in Blutabstrichen | |
| | | TB | (n=26) | Die Diagnose erfolgte durch den Nachweis von AFB der TB im Sputumabstrich | |
| Deutschland | 10 | NEC | (n=10) | Nicht endemische gesunde Freiwillige aus Deutschland | |

Die diagnostische Leistung der rK39, KLO8 und rKLI8,3-Testantigene ist in Tabelle 2 dargestellt.

**Tabelle 2:Diagnostische Leistung der Testantigene rK39, KLO8 und rKLi8.3 im ELISA für die viszerale Leishmaniose (VL) im Sudan: AUC= area under curve; TP= true positives; FN = false negatives; TN = true negatives; FP = false positives;**

| **Testantigen (ELISA)** | **Cut off Wert** | **AUC** | **TP** | **FN** | **TN** | **FP** | **Sensitivität %** | **Spezifität %** | **PPV%** | **NPV %** | **DEV%** |
|---|---|---|---|---|---|---|---|---|---|---|---|
| **rK39** | 0.146 | 0.9678 | 157 | 15 | 118 | 8 | 91.28 | 93.65 | 95.15 | 88.72 | 92.28 |
| **KLO8** | 0.124 | 0.9811 | 159 | 13 | 123 | 3 | 92.44 | 97.62 | 98.15 | 90.44 | 94.63 |
| **rKLi8.3** | 0.106 | 0.9927 | 167 | 5 | 125 | 1 | 97.10 | 99.20 | 99.40 | 96.15 | 97.98 |

PPV = positive predictive value; NPV = negative predictive value; DEV = diagnostic efficiency value. Die Spezifität wurde anhand von 126 viszeralen Leishmaniose-negativen Seren berechnet, davon 85 endemische Kontrollseren, 10 nichtendemische Kontrollseren, 5 Malaria-Seren und 26 Tuberkulose-Seren. Die Sensitivität wurde anhand von 172 viszeralen Leishmaniose-Seren berechnet. Die Werte für Sensitivität, Spezifität, PPV, NPV und DEV wurden mit einem Konfidenzintervall von 95 % berechnet. Die Seren wurden mit einer Verdünnung von 1:800 und die Test-antigene mit der Konzentration von 5 ng verwendet. Der Cut-off-Wert wurde als mittlerer Absorptionswert der endemischen Kontrollseren + 3 Standardabweichungen festgelegt.

Die quantitative Analyse des Testantigens rKLi8.3 mit viszeraler Leishmaniose (VL) Patientenseren zeigte deutlich höhere Antikörperspiegel als bei den Kontrollpersonen. Die mit rKLi8.3 Testantigen getesteten Seren zeigten höhere OD-Werte gegenüber denen, die mit den Testantigenen rK39 und KLO8 getestet wurden. Die Reaktivität der Antikörper (IgG) gegen rKLi8.3 zeigte keine Kreuzreaktivität mit Malaria und Tuberkulose. Im Gegensatz dazu zeigte der KLO8 und rK39-ELISA eine geringere Spezifität und Sensitivität bei der Diagnose von VL, sowie eine gleichzeitig erhöhte Kreuzreaktivität mit Malaria und Tuberkulose. Die Kreuzreaktivität von rK39 mit Tuberkulose- und Malaria spezifischen Antikörpern ist auch publiziert worden.

Die vorliegenden Daten weisen eindeutig darauf hin, dass das erfindungsgemäße rekombinante rKLi8.3-Testantigen für die Diagnose der viszeralen Leishmaniose bei Patienten aus dem Sudan eine deutlich höhere Sensitivität und Spezifität als die bekannten Testantigene aufweist.

Um die Leistung des erfindungsgemäßen Testantigens auch für Patienten außerhalb des Sudans zu testen, wurden zusätzlich Seren aus Indien in einem ELISA mit rKLi8.3-, KLO8- und rK39 Antigenen getestet. In Tabelle 3 sind alle getesteten Seren mit ihrer Herkunft und ihren Eigenschaften aufgelistet. Die Ergebnisse dazu sind in Tabelle 4 dargestellt. Das rKLi8.3-Testantigen reagierte mit allen VL-Patienten aus Indien (19 von 19, d.h. 100 %).

**Tabelle 3: Beschreibung der Seren von Patienten mit viszeraler Leishmaniose und Kontrollen aus Indien. Abkürzungen: VL, viszerale Leishmaniose; EC, endemische Kontrollen; MA, Malarial; TX, Toxoplasmose.**

| **Herkunft (Land)** | **Nr. von Seren** | **Klinischer Fall** | | **Eigenschaften** | **Klassifizierung** |
|---|---|---|---|---|---|
| **Indien** | 30 | VL | (n=19) | Die Diagnose basiert auf dem Nachweis von Parasiten in gefärbten Lymphknotenabstrichen | bestätigte VL Patienten |
| | | EC | (n=9) | Aus dem gleichen VL-Endemiegebiet (Indien) | Negativkontrollen |
| | | MA | (n=1) | Die Diagnose erfolgte durch den Nachweis von Malaria Parasiten in Blutabstrichen. | |
| | | TX | (n=1) | Die Diagnose erfolgte durch Nachweis von Toxoplasmose spezifischen Antikörpern | |

**Tabelle 4; Diagnostische Leistung von rK39-, KLO8- und rKLi8.3-Testantigen (Elisa) für viszerale Leishmaniose in Indien: AUC= Fläche unter der Kurve; TP=korrekt positive; FN = falsch negative; TN = korrekt negativ; FP = falsch positiv; PPV = positive Vorhersage; NPV = negative Vorhersage; DEV = diagnostische Effizienz.**

| **Testantigen (ELISA)** | **Cut off Wert** | **AUC** | **TP** | **FN** | **TN** | **FP** | **Sensitivität %** | **Spezifität %** | **PPV %** | **NPV %** | **DEV %** |
|---|---|---|---|---|---|---|---|---|---|---|---|
| **rK39** | 0.146 | 0.9809 | 18 | 1 | 10 | 1 | 94.73 | 90.91 | 94.73 | 90.91 | 93.33 |
| **KLO8** | 0.124 | 0.9856 | 18 | 1 | 10 | 1 | 94.73 | 90.91 | 94.73 | 90.91 | 93.33 |
| **rKLi8.3** | 0.106 | 0.9904 | 19 | 0 | 10 | 1 | 100 | 90.91 | 95 | 100 | 96.66 |

Die Spezifität wurde anhand von 11 Kontrollseren (viszerale Leishmaniosenegative) berechnet, zu denen 9 endemische Kontrollseren, 1 Malaria-Serum und 1 Toxoplasmose-Serum gehörten. Die Sensitivität wurde anhand von 19 viszeraler Leishmaniose-Seren berechnet. Die Werte für Sensitivität, Spezifität, PPV, NPV und DEV wurden mit einem Konfidenzintervall von 95 % berechnet. Die Seren wurden mit Verdünnungen von 1:800 und Proteinkonzentrationen von 5 ng getestet. Der Cut-off-Wert wurde als mittlerer Absorptionswert der endemischen Kontrollseren + 3 Standardabweichungen festgelegt.

Um die Leistung des erfindungsgemäßen Testantigens auch an Hunden zu testen, wurden 332 Seren von Hunden mit caniner viszeralen Leishmaniose (CVL) aus Kroatien in einem ELISA mit rKLi8.3-, KLO8- und rK39-Testantigenen getestet. Die Infektion der Hunde (mit *L. infantum*) wurde durch mikroskopische Analyse von Knochenmarkaspiraten oder IFAT bestätigt.

Zur Bestimmung der Eignung und Effizienz des Testantigens rKLi8.3 für den Nachweis von *Leishmania*-Antikörpern bei der Hundeleishmaniose wurden 183 Seren aus Kroatien analysiert. Darüber hinaus wurden 149 Seren nicht infizierter, gesunder Hunde getestet, um den Cut-off-Wert zu bestimmen, der bei 0,11 für rKLi8.3 liegt (siehe Tabelle 5). Der ELISA wurde unter Verwendung der gleichen Konzentration des Testantigens (5 ng/Well) und den gleichen Serumverdünnungen (1:800) optimiert und durchgeführt. Der ELISA zur Analyse von Hundeseren wurde wie oben beschrieben durchgeführt, mit Ausnahme des Sekundärantikörpers, da hier Kaninchen-Anti-Hund-IgG (H+L) (Jackson Immunoresearch Laboratories, Inc.) verwendet wurde. Die Ergebnisse sind in Tabelle 6 dargestellt. Wie bereits für den Menschen gezeigt, eignet sich das rKLi8.3-Testantigen auch hervorragend für die Serodiagnostik Leishmanien-infizierter Hunde.

**Tabelle 5: Beschreibung der Seren von Hunden mit caniner viszeraler Leishmaniose und Kontrollen aus Kroatien. Abkürzungen: CVL, canine viszerale Leishmaniose; EC, endemische Kontrollen; IFAT, indirekter Immunfluoreszenz-Antikörper-Test.**

| **Herkunft (Land)** | **Nr. von Seren** | **Klinischer Fall** | | **Eigenschaften** | **Klassifizierung** |
|---|---|---|---|---|---|
| **Kroatien** | 332 | CVL | (n=183) | IFAT (Indirekter Immunfluoreszenz-Antikörper-Test) | bestätigte CVL Hunde |
| | | EC | (n=149) | Aus dem gleichen VL-Endemiegebiet (Kroatien) | Negativkontrollen |

**Tabelle 6: Diagnostische Leistung (ELISA) von rK39-, KLO8- und rkLi8.3-Testantigenen bei Hunden mit viszeraler Leishmaniose (CVL). Die Spezifität wurde anhand von 149 viszeralen Leishmaniose-negativen Seren aus dem endemischen Gebiet berechnet. Die Sensitivität wurde anhand von 183 viszeralen Leishmaniosepositiven Seren berechnet. Die Werte für Sensitivität, Spezifität, PPV, NPV und DEV wurden mit einem Konfidenzintervall von 95 % berechnet. Die Seren wurden in der Verdünnung von 1:800 und Proteinkonzentration von 5 ng getestet. Der Cut-off-Wert wurde als mittlerer Absorptionswert der endemischen Kontrollseren + 3 Standardabweichungen festgelegt. Abkürzungen: CVL, canine viszerale Leishmaniose; EC, endemische Kontrollen. AUC = Fläche unter der Kurve; TP = korrekt positive; FN = falsch negative; TN = korrekt negativ; FP = falsch positiv; PPV = positive Vorhersage; NPV = negative Vorhersage; DEV = diagnostische Effizienz.**

| **Testantigen EUSA** | **Cut off Wert** | **AUC** | **TP** | **FN** | **TN** | **FP** | **Sensitivität%** | **Spezifität %** | **PPV %** | **NPV%** | **DEV%** |
|---|---|---|---|---|---|---|---|---|---|---|---|
| **KLO8** | 0.14 | 0.9903 | 180 | 3 | 135 | 14 | 98,3 | 90,6 | 92,78 | 97,82 | 94,87 |
| **rK39** | 0.15 | 0.9901 | 180 | 3 | 136 | 13 | 98,3 | 91,3 | 93,26 | 97,84 | 95,18 |
| **rKLi8.3** | 0.11 | 0.9922 | 181 | 2 | 143 | 6 | 98,9 | 96 | 96,79 | 98,62 | 97,59 |

Im Vergleich zu dem derzeit am meisten verwendeten rK39-Testantigen trägt das rKLi8.3-Testantigen zu einer verbesserten Serodiagnostik der viszeralen Leishmanieninfektion bei Menschen und Hunden bei und kann damit auch genauere Informationen über das epidemiologische Ausmaß und deren Kontrollmaßnahmen liefern. Neben der verbesserten diagnostischen Leistung (Sensitivität und Spezifität) bietet die auf rKLi8.3 basierte Serodiagnostik auch den Vorteil der Unabhängigkeit vom Erregerstamm (einsetzbar in allen VL-Endemiegebieten) und der infizierten Spezies, wodurch sie für Mensch und Hund geeignet ist.

Die Verwendung des erfindungsgemäßen Testantigens rKLi8.3 führt zu einer verbesserten Diagnose der viszeralen Leishmaniose und ist damit eine effiziente und kostengünstige Methode zur Überwachung und Kontrolle der Ausbreitung dieser Infektion.

Das erfindungsgemäße Testantigen wurde lyophilisiert und in tiefgefrorenem Zustand aufbewahrt. Dabei konnten keine wesentlichen Qualitätsverluste nach einer Lagerungsdauer von zweieinhalb Jahren festgestellt werden. Bei einer Lagerungsdauer des lyophilisierten erfindungsgemäßen Testantigens über mindestens fünf Jahre ist die Lagerstabilität möglich.

### Abbildungen

**Abbildung 1** zeigt die Expression, Aufreinigung und Western-Blot-Analyse des rekombinanten erfindungsgemäßen Testantigens (Proteins) rKLi8.3. Das rekombinante erfindungsgemäße Testantigen rKLi8.3 erkennt das positive Patientenserum, während das negative Serum nicht mit dem rekombinanten erfindungsgemäßen Testantigen (Protein) rKLi8.3 reagiert (Spur 1).

**Abbildung 2** zeigt die Ergebnisse des indirekten IgG-ELISAs zum spezifischen Nachweis von VL. Zur Bestimmung der optimalen ELISA-Bedingungen wurden 10 gepoolte VL-Seren oder 10 gepoolte gesunde Kontrollseren in serieller Zweifachverdünnung (1:25-1:25600) gegen verschiedene Konzentrationen des rekombinanten Testantigens rKLi8.3 titriert. A: 50 ng/100 µl, B: 25 ng/100 µl, C: 10 ng/100 µl, D: 5 ng/100 µl. Die Seren wurden in Doppelbestimmung getestet und die Mittelwerte wurden berechnet.

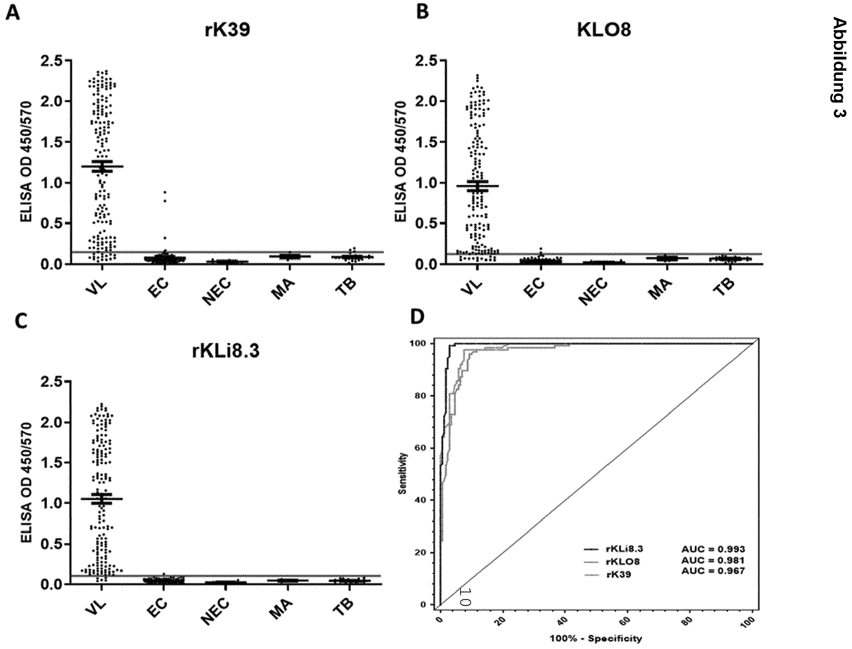

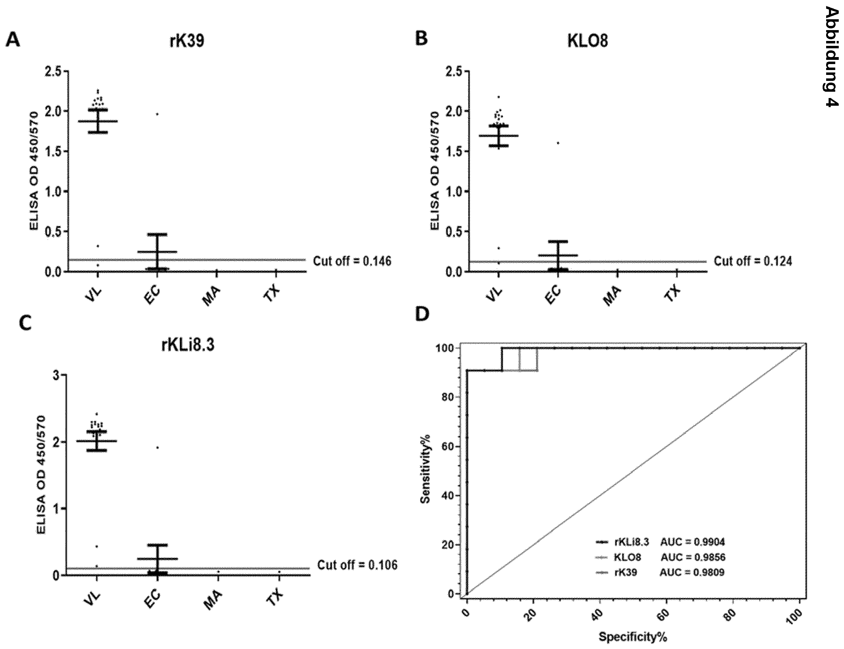

**Abbildung 3** zeigt die Antikörperreaktion humaner Seren (Sudan, Afrika) mit A: rK39, B: KLO8 und C: rKLi8.3. ELISA-Platten wurden mit rekombinanten Proteinen gecoated (5 ng/100 µl in 0.1M Natriumcarbonat); Antikörperbindung von Patienten mit viszeraler Leishmaniose (VL) (•, n = 172) und verschiedener Kontrollseren wurde verglichen. EC: Kontrollen aus Endemiegebieten [n = 126]; NEC: Kontrollen aus Nicht-Endemiegebieten [n = 10]; MA: Malaria-Patienten [n = 5]; TB: Tuberkulose-Patienten [n = 26]. Die individuellen OD-Werte sind als Dots dargestellt. Die Seren wurden in einer 1:800 Verdünnung getestet. • repräsentiert Proben jenseits des Cut-off Bereichs, FN in der VL Gruppe und FP in der HC Gruppe. Die Mittellinien repräsentieren die Mittelwerte jeder Gruppe. D: Vergleich der ROC-Kurven der rekombinanten Proteine. Die ROC-Kurven für rKLi8.3-, rK39- und KLO8-ELISA wurden generiert anhand der Prism 9.0 Software und zur Ermittlung der Sensitivität, Spezifität und AUC für jedes Assay verwendet. Die y-Achse repräsentiert die Sensitivität und die x-Achse die Spezifität für jedes Assay. **Abbildung 4** zeigt A: rK39-, B: KLO8- und C: rKLi8.3-ELISA Ergebnisse mit Patienten- und Kontrollseren aus Indien. Die Reaktivität wurde mit Seren von VL-Patienten (n=19) oder Gesunden (n=9), Malaria (n=1) und Toxoplasmose (n=1) überprüft. D: Vergleich der ROC-Kurven der rekombinanten Testantigene. Die ROC-Kurven für rKLi8.3-, rK39- und KLO8-ELISA wurden generiert anhand der Prism 9.0 Software und zur Ermittlung der Sensitivität, Spezifität und AUC für jedes Assay verwendet. Die y-Achse repräsentiert die Sensitivität und die x-Achse die Spezifität für jedes Assay.

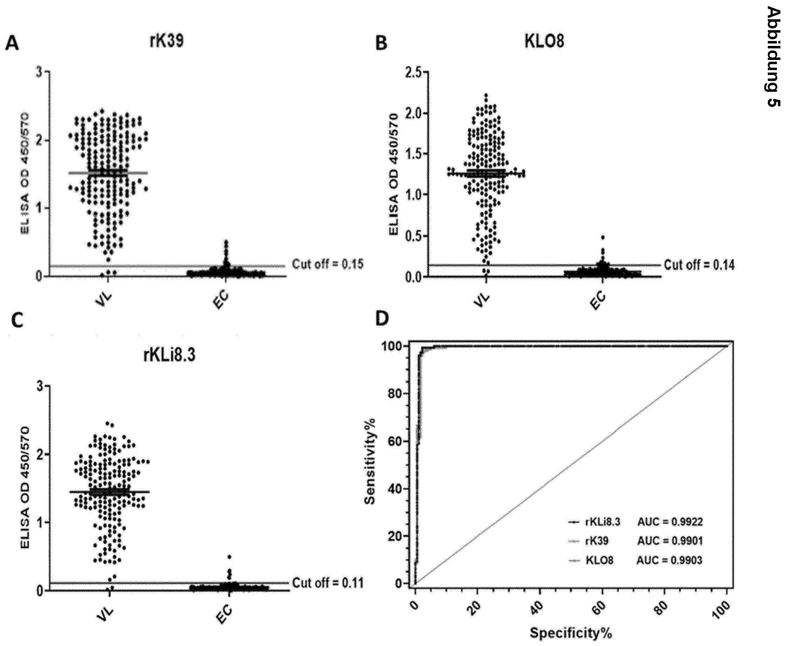

**Abbildung 5** zeigt Vergleich der Reaktivität von Hundeseren aus Kroatien und ROC-Kurven. A: rK39-, B: KLO8- und C: rKLi8.3- ELISA Ergebnisse mit Hunde- und Kontrollseren aus Kroatien. Die Reaktivität wurde mit Seren von CVL-Hunden (n=183) oder Gesunden (n=149) überprüft. D: In den ROC-Kurven wird die Sensitivität jedes Tests durch die y-Achse und die Spezifität durch die x-Achse dargestellt. Die ROC-Kurven wurden mit der Software Prism 9.0 erstellt und verwendet, um Cut-off, Sensitivität, Spezifität und AUC (Fläche unter der Kurve) für jeden Assay zu bestimmen.

**Abbildung 6** zeigt zusammengefasst die Kinesin-Polymorphismen von *L*. *donovani, L. infantum* und *L*. *archibaldi* aus Ostafrika und *L*. *donovani* aus Indien (KE16) sowie die Abweichungen zwischen den beiden geografischen Regionen im Vergleich zu dem von *L*. *infantum* (*L. chagasi*) rK39 aus Brasilien.

rK39 stammt von *L*. *infantum* (*L. chagasi*)*,* KE16 stammt von *L*. *donovani,* KLO8 ist von *L*. *donovani* und P = Prä-Repeat-Region.

### Sequenzen

**SEQ ID NO. 1: Sequenz des Forward-Primers**
   5'-GAGCTCGCAACCGAGTGGGAGG-3'
**SEQ ID NO. 2: Sequenz des Reverse-Primers**
   5'- GCTCCGCAGCGCGCTCC-3'
**SEQ ID NO. 3: DNA-Sequenz KLi8.3** 
GAGCTCGCAACCGAGTGGGAGGACGCACTCCGCGAGCGTGCGCTTGCAGA
GCGTGACGAAGCCGCTGCAGCCGAACTTGATGCCGCAGCCTCTACTTCCCA
AAACGCACGTGAAAGCGCCTCCGAGCGGCTAACCAGCCTTGAGCAGCTGCT
TCGCGAATCCGAGGAGCGCGCTGCGGAGCTGGCGAGTCAGCTGGAGTCCA
CTACTGCTGCGAAGATGTCGGCGGAGCAGGACCGCGAGAACACGAGGGCCA
CGCTAGAGCAGCAGCTTCGTGACTCCGAGGAGCGCGCTGCGGAGCTGGCG
AGCCAGCTGGAGGCCACTGCTGCTGCGAAGTCGTCGGCGGAGCAGGACCG
CGAGAACACGAGGGCCGCGTTGGAGCAGCTGCTTCGCGAATCCGAGGAGC
GCGCTGCGGAGCTGGCGAGCCAGCTGGAGGCCACTGCTGCTGCGAAGATG
TCGGCGGAGCAGGACCGCGAGAACACGAGGGCCGCGTTGGAGCAGCAGCT
TCGTGACTCCGAGGAGCGCGCTGCGGAGCTGGCGAGTCAGCTGGAGTCCAC
TACTGCTGCGAAGATGTCGGCGGAGCAGGACCGCGAGAACACGAGGGCCAC
GCTAGAGCAGCAGCTTCGTGACTCCGAGGAGCGCGCTGCGGAGCTGGCGA
GTCAGCTGGAGTCCACTACTGCTGCGAAGACGTCGGCGGAGCAGGACCGCG
AGAACACGAGGGCCGCGTTGGAGCAGCAGCTTCGTGACTCCGAGGAGCGC
GCTGCGGAGCTGGCGAGCCAGCTGGAGGCCACTGCTGCTGCGAAGTCGTC
GGCGGAGCAGGACCGCGAGAACACGAGGGCCGCGTTGGAGCAGCAGCTTC
GTGACTCCGAGGAGCGCGCTGCGGAGCTGGCGAGCCAGCTGGAGGCCACT
GCTGCTGCGAAGTCGTCGGCGGAGCAGGACCGCGAGAACACGAGGGCCAC
GCTAGAGCAGCAGCTTCGTGACTCCGAGGAGCGCGCTGCGGAGCTGGCGA
GTCAGCTGGAGTCCACTACTGCTGCGAAGATGTCGGCGGAGCAGGACCGCG
AGAACACGAGGGCCACGCTAGAGCAGCAGCTTCGTGACTCCGAGGAGCGCG
CTGCGGAGC
**SEQ ID NO. 4: Aminosäuren-Repeat-Sequenz KLi8.3** 
LEQLLRESEERAAELASQLESTTAAKMSAEQDRENTRAT
LEQQLRDSEERAAELASQLEATAAAKSSAEQDRENTRAA
LEQLLRESEERAAELASQLEATAAAKMSAEQDRENTRAA
LEQQLRDSEERAAELASQLESTTAAKMSAEQDRENTRAT
LEQQLRDSEERAAELASQLESTTAAKTSAEQDRENTRAA
LEQQLRDSEERAAELASQLEATAAAKSSAEQDRENTRAA
LEQQLRDSEERAAELASQLEATAAAKSSAEQDRENTRAT
LEQQLRDSEERAAELASQLESTTAAKMSAEQDRENTRAT
LEQQLRDSEERAAE
**SEQ ID NO. 5: Sequenz des Forward-Primers für Subklonierung**
   5'-GTACATATGGAGCTCGCAACCGAGTGGGAGGACGCA-3'
**SEQ ID NO. 6: Sequenz des Reverse-Primers für Subklonierung**
   5'-TACCTCGAGCAGTGTGCTGGAATTCGCCCTTACTCCGCAGC-3'
**SEQ ID NO. 7: Nukleotid-Sequenz von rekombinantem 6xhis-KLi8.3** 
ATGGGCAGCAGCCATCATCATCATCATCACAGCAGCGGCCTGGTGCCGCGC
GGCAGCCATATGGAGCTCGCAACCGAGTGGGAGGACGCACTCCGCGAGCGT
GCGCTTGCAGAGCGTGACGAAGCCGCTGCAGCCGAACTTGATGCCGCAGCC
TCTACTTCCCAAAACGCACGTGAAAGCGCCTCCGAGCGGCTAACCAGCCTTG
AGCAGCTGCTTCGCGAATCCGAGGAGCGCGCTGCGGAGCTGGCGAGTCAG
CTGGAGTCCACTACTGCTGCGAAGATGTCGGCGGAGCAGGACCGCGAGAAC
ACGAGGGCCACGCTAGAGCAGCAGCTTCGTGACTCCGAGGAGCGCGCTGC
GGAGCTGGCGAGCCAGCTGGAGGCCACTGCTGCTGCGAAGTCGTCGGCGG
AGCAGGACCGCGAGAACACGAGGGCCGCGTTGGAGCAGCTGCTTCGCGAAT
CCGAGGAGCGCGCTGCGGAGCTGGCGAGCCAGCTGGAGGCCACTGCTGCT
GCGAAGATGTCGGCGGAGCAGGACCGCGAGAACACGAGGGCCGCGTTGGA
GCAGCAGCTTCGTGACTCCGAGGAGCGCGCTGCGGAGCTGGCGAGTCAGCT
GGAGTCCACTACTGCTGCGAAGATGTCGGCGGAGCAGGACCGCGAGAACAC
GAGGGCCACGCTAGAGCAGCAGCTTCGTGACTCCGAGGAGCGCGCTGCGG
 
AGCTGGCGAGTCAGCTGGAGTCCACTACTGCTGCGAAGACGTCGGCGGAGC
AGGACCGCGAGAACACGAGGGCCGCGTTGGAGCAGCAGCTTCGTGACTCCG
AGGAGCGCGCTGCGGAGCTGGCGAGCCAGCTGGAGGCCACTGCTGCTGCG
AAGTCGTCGGCGGAGCAGGACCGCGAGAACACGAGGGCCGCGTTGGAGCA
GCAGCTTCGTGACTCCGAGGAGCGCGCTGCGGAGCTGGCGAGCCAGCTGG
AGGCCACTGCTGCTGCGAAGTCGTCGGCGGAGCAGGACCGCGAGAACACG
AGGGCCACGCTAGAGCAGCAGCTTCGTGACTCCGAGGAGCGCGCTGCGGA
GCTGGCGAGTCAGCTGGAGTCCACTACTGCTGCGAAGATGTCGGCGGAGCA
GGACCGCGAGAACACGAGGGCCACGCTAGAGCAGCAGCTTCGTGACTCCGA
GGAGCGCGCTGCGGAGTAA
**SEQ ID NO. 8: Aminosäuren-Sequenz von rekombinantem 6xhis-rKLi8.3**

MGSSHHHHHHSSGLVPRGSHMELATEWEDALRERALAERDEAAAAELDAAAST
SQNARESASERLTSLEQLLRESEERAAELASQLESTTAAKMSAEQDRENTRATL
EQQLRDSEERAAELASQLEATAAAKSSAEQDRENTRAALEQLLRESEERAAELA
SQLEATAAAKMSAEQDRENTRAALEQQLRDSEERAAELASQLESTTAAKMSAE
QDRENTRATLEQQLRDSEERAAELASQLESTTAAKTSAEQDRENTRAALEQQLR
DSEERAAELASQLEATAAAKSSAEQDRENTRAALEQQLRDSEERAAELASQLEA
TAAAKSSAEQDRENTRATLEQQLRDSEERAAELASQLESTTAAKMSAEQDRENT
RATLEQQLRDSEERAAE

## Patentansprüche

1. Verfahren zum Nachweis von Antikörpern gegen Leishmanien in einer biologischen Probe, umfassend die Schritte:
(i) Bereitstellung einer biologischen Probe eines Menschen oder eines Hundes;
(ii) Bereitstellung des auf einer Unterlage gebundenen Testantigens mit der SEQ ID NO. 8, das 8,3 repetitive Sequenzen aufweist und das von Antikörpern gegen verschiedene Leishmanienstämme erkannt und gebunden werden kann;
(iii) Inkubation der biologischen Probe aus Schritt (i) mit dem Testantigen aus Schritt (ii), so dass Antigen-Antikörper-Komplexe gebildet werden, wenn in der biologischen Probe aus Schritt (i) Antikörper gegen mindestens einen Leishmanienstamm enthalten sind;
(iv) Entfernung von nicht an das Testantigen aus Schritt (ii) gebundene Antikörper und sonstigem Material aus der biologischen Probe aus Schritt (i) in einem ersten Waschschritt;
(v) Zugabe eines human-spezifischen Sekundärantikörpers, wenn die biologische Probe aus Schritt (i) von einem Mensch stammt oder Zugabe eines canin-spezifischen Sekundärantikörpers, wenn die biologische Probe aus Schritt (i) von einem Hund stammt, wobei der jeweilige Sekundärantikörper durch ein Mittel zur Sichtbarmachung einer Bindung des Sekundärantikörpers nachweisbar ist, so dass der jeweilige Sekundärantikörper an die Antigen-Antikörper-Komplexe aus Schritt (iii) bindet;
(vi) Entfernung von nicht gebundenen Sekundärantikörpern aus Schritt (v) in einem zweiten Waschschritt;
(vii) Sichtbarmachung der Bindung des Sekundärantikörpers an den Antigen-Antikörper-Komplexen aus Schritt (iii) durch Zugabe eines geeigneten Mittels.

2. Ein Verfahren nach Anspruch 1, **dadurch gekennzeichnet, dass** die biologische Probe Serum, Plasma, Vollblut, Lymphknotenaspirat, Speichel, Gewebsflüssigkeit, Liquor cerebrospinalis, Urin oder eine andere körpereigene Flüssigkeit ist.

3. Ein Verfahren nach Anspruch 1, **dadurch gekennzeichnet, dass** das Mittel zum Nachweis der Bindung zwischen dem Sekundärantikörper und den Antigen-Antikörper-Komplexen aus Schritt (v) ein Enzym ist, welches mit einem geeigneten Substrat eine Farbveränderung bewirkt.

4. Ein Verfahren nach Anspruch 1, **dadurch gekennzeichnet, dass** das Mittel zum Nachweis der Bindung zwischen dem Sekundärantikörper und den Antigen-Antikörper-Komplexen aus Schritt (v) eine Farbreaktion auslöst.

5. Ein Verfahren nach Anspruch 1, **dadurch gekennzeichnet, dass** das Mittel zum Nachweis der Bindung zwischen dem Sekundärantikörper und den Antigen-Antikörper-Komplexen aus Schritt (v) Goldkolloid ist.

6. Ein Verfahren nach Anspruch 1, **dadurch gekennzeichnet, dass** Antikörper gegen Leishmanien nachgewiesen werden, ausgewählt aus der Gruppe *Leishmania chagasi, Leishmania donovani* und *Leishmania infantum.*

7. Ein Verfahren nach Anspruch 1, **dadurch gekennzeichnet, dass** die Spezifität mindestens 90 % ist und Sensitivität mindestens 97% ist.

8. Ein Testkit zur Durchführung des Verfahrens nach einem der vorhergehenden Ansprüche, **dadurch gekennzeichnet, dass** er zumindest aufweist:
- eine Unterlage, die geeignet ist, das Testantigen zu binden;
- ein auf dieser Unterlage gebundenes Testantigen mit der SEQ ID NO. 8, das 8,3 repetitive Sequenzen aufweist und das von Antikörpern gegen verschiedene Leishmanienstämme erkannt und gebunden werden kann und Antigen-Antikörper-Komplexe bildet;
- einen human-spezifischen Sekundärantikörper, wenn die biologische Probe von einem Mensch stammt oder einen canin-spezifischen Sekundärantikörper, wenn die biologische Probe von einem Hund stammt;
- ein Mittel zum Nachweis der Bindung zwischen dem Sekundärantikörper und den Antigen-Antikörper-Komplexen.

9. Ein Testkit nach Anspruch 8, **dadurch gekennzeichnet, dass** die Unterlage eine Mikrotiterplatte, ein Testfeld oder ein Teststreifen ist.

10. Herstellung des Testantigens gemäß der SEQ ID NO. 8, das 8,3 repetitive Sequenzen aufweist und das von Antikörpern gegen verschiedene Leishmanienstämme erkannt und gebunden werden kann, umfassend die Schritte:
(i) Bereitstellen von promastigoten Leishmanien;
(ii) Isolieren der genomischen DNA aus den promastigoten Leishmanien aus Schritt (i);
(iii) Amplifizieren des Genabschnitt aus der DNA aus Schritt (ii) gemäß SEQ ID NO. 3 mit dem Forward-PCR-Primer gemäß der SEQ ID NO. 1 und dem Reverse-PCR-Primer gemäß der SEQ ID NO. 2, so dass ein Amplifikationsprodukt entsteht;
(iv) Isolation und Aufreinigung des Amplifikationsproduktes aus Schritt (iii);
(v) Klonierung des aufgereinigten Amplifikationsproduktes aus Schritt (iv) in einen Vektor;
(vi) Transfektion von kompetenten E. *coli* mit dem Vektor aus Schritt (v);
(vii) Expression des Testantigens in E. *coli*;
(viii) Lyse der E. *coli* aus Schritt (vii);
(ix) Isolation des Testantigens aus den lysierten E. *coli* aus Schritt (viii);
(x) Aufreinigung des Testantigens aus Schritt (ix).

11. Testantigen mit der SEQ ID NO. 8 hergestellt nach dem Verfahren gemäß Anspruch 10.

12. Testantigen gemäß Anspruch 10, **dadurch gekennzeichnet, dass** es in lyophilisierter Form eine Lagerstabilität von mindestens zweieinhalb Jahren aufweist.

13. Testantigen gemäß Anspruch 10, **dadurch gekennzeichnet, dass** es in lyophilisierter Form eine Lagerstabilität von mindestens fünf Jahren aufweist.

14. Verwendung des Testantigens gemäß Anspruch 10 in einem Verfahren gemäß Anspruch 1.

15. Verwendung des Testantigens gemäß Anspruch 10 in einem Testkit gemäß Anspruch 8.
